# EUROPEAN PATENT SPECIFICATION

(11) **EP 1 414 453 B1**
(45) Date of publication and mention of the grant of the patent: **19.03.2008**
(21) Application number: 02751479.3
(22) Date of filing: 29.07.2002
(51) Int. Cl.: A61K 31/445, A61P 37/08, C07D 211/22

(54) **FEXOFENADINE HYDROCHLORIDE POLYMORPH**
FEXOFENADINE HYDROCHLORID POLYMORPH
AGENT POLYMORPHE DE HYDROCHLORURE DE FEXOFENADINE

(30) Priority: 31.07.2001 EP 01610081
(43) Date of publication of application: 06.05.2004
(73) Proprietor: Texcontor Etablissement, 9490 Vaduz (LI)
(72) Inventor: MILLA, Frederico Junquera, 50015 Zaragoza (ES)
(74) Representative: Plougmann & Vingtoft A/S
(86) International application number: PCT/IB2002/002954
(87) International publication number: WO 2003/011295

(56) References cited:
- WO-A-95/31437

## Description

### FIELD OF THE INVENTION

This application relates to a new polymorph of fexofenadine and to a process for the preparation thereof.

### BACKGROUND OF THE INVENTION

Terfenadine, 1-(p-tert-butylphenyl)-4-[4'-(alpha-hydroxydiphenylmethyl)-1'-piperidenyl]-butanol, is a non-sedating anti-histamine. It is known to be a specific H₂-receptor antagonist that is also devoid of any anticholingeric, antiserotoninergic and antiadrenergic effects both *in vivo* and *in vitro.*

However, terfenadine has been linked to potentially fatal abnormal heart rhythms in some patients with liver disease or who also take the antifungal drug ketoconazole or the antibiotic erythromycin.

In animal and human metabolic studies, terfenadine was shown to undergo high first-pass effect, which results in readily measurable plasma concentrations of the major metabolite 4-[4[4-(hydroxy diphenyl methyl)-1-piperidenyl]-1-hydroxy butyl]-α,α-dimethylphenyl acetic acid, also known as terfenadine carboxylic acid metabolite or fexofenadine, the structure of which is illustrated below.

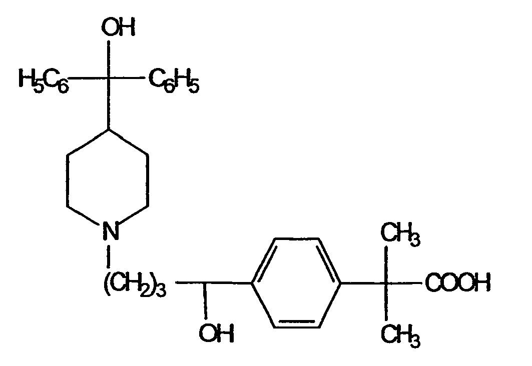

Fexofenadine possesses anti-histamine activity in animal models and is believed to lack the cardiac side effects seen with terfenadine. Moreover, it has been postulated that terfenadine is in fact a pro-drug and fexofenadine is the active agent.

There has therefore been considerable interest in preparing fexofenadine since its use may eliminate a number of the side effects associated with the use of terfenadine.

Fexofenadine hydrochloride is an effective antihistamine which avoids adverse effects associated with the administration of terfenadine.

The pharmaceutical industry has, of late, conducted studies on polymorphism in drugs and the difference in the activity of different polymorphic forms of a given drug. The term polymorphism is meant to include different physical forms, crystal forms, crystalline/liquid crystalline/non-crystalline (amorphism) forms. Polymorphism has interested scientists after observations were made that many antibiotics, antibacterials, tranquillisers, etc. exhibit polymorphism and some/one of the polymorphic forms of a given drug exhibits superior bioavailability and consequently shows much higher activity compared to other polymorphs. It has also been disclosed that some amorphic forms of a number of drugs exhibit different dissolution characteristics and in some cases different bioavailability patterns compared to the crystalline form. For some therapeutic indications one bioavailability pattern may be favoured over another.

Polymorphic forms of fexofenadine have been identified in the prior art. In WO 00/71124 there is described an amorphic form of fexofenadine hydrochloride prepared by dissolving crystalline fexofenadine hydrochloride in a suitable solvent or dissolving fexofenadine base in a suitable solvent and adding a suitable solvent containing hydrogen chloride and recovering the amorphic form of fexofenadine hydrochloride from the solution thereof by spray drying of freeze drying techniques.

WO 95/31437 also describes polymorphic forms of fexofenadine, in particular so-called anhydro form I and form III of fexofenadine hydrochloride and hydrated forms II and IV of fexofenadine hydrochloride. The Form I polymorph is perhaps the closest in structure to the polymorph of the present invention, nevertheless, the Form I polymorph exhibits a very different X-ray diffraction spectrum than that required below.

### DESCRIPTION OF THE INVENTION

The present inventors have surprisingly found a new polymorphic form of fexofenadine hydrochloride which may be isolated using pentanone. It is envisaged that the polymorphic form of fexofenadine hydrochloride described herein will have particularly beneficial medicinal properties, be especially bioavailable and have a long shelf life.

Thus, the invention provides a fexofenadine hydrochloride polymorph having the following X-ray powder diffraction pattern obtained using K_{α}1 Cu(λ=1.5406Å) radiation

| D-space, Angstroms |
|---|
| 12.25 |
| 11.28 |
| 8.78 |
| 8.17 |
| 7.73 |
| 6.77 |
| 6.32 |
| 6.11 |
| 5.61 |
| 5.32 |
| 5.13 |
| 4.98 |
| 4.88 |
| 4.85 |
| 4.32 |
| 3.87 |
| 3.64 |
| 3.49 |
| 3.38 |
| 3.23 |
| 2.91 |
| 2.80 |

The invention also provides a pharmaceutical composition comprising a fexofenadine hydrochloride polymorph as hereinbefore described along with one or more pharmaceutical carriers/excipients.

The invention further provides a fexofenadine hydrochloride polymorph as hereinbefore described for use in medicine, e.g. as a antihistamine, antiallergy agent or bronchodilator.

The invention still further provides the use of a fexofenadine hydrochloride polymorph as hereinbefore described in the manufacture of an medicament for use as an antihistamine, antiallergy agent or bronchodilator.

The water content (measured by the Karl Fischer water determination method) of the fexofenadine hydrochloride polymorph of the invention should be below 0.5%, e.g. between 0.1 to 0.4%, preferably below 0.3% e.g. 0.2 to 0.3%.

The melting point of the fexofenadine hydrochloride polymorph (as measured by differential scanning calorimetry) should be in the range: melt endotherm onset 195 to 197°C.

The infrared spectrum of the fexofenadine hydrochloride polymorph has also been obtained and is described below. The underlined peaks are considered the most characteristics of the polymorph.

IR νₘₐₓ(cm⁻¹)(KBr): 3412, 3051, 2980, 2940, 2871, 2725, 1713, 1512, 1492, 1468, 1447, 1389, 1273, 1250, 1238, 1169, 1150, 1099, 1091, 1066, 1020, 1009, 1000, 967, 881, 841, 819, 751, 744, 704, 693, 664, 637.

Thus, the invention also provides a fexofenadine polymorph having the characteristic IR peaks described above.

The X-ray diffraction pattern of the polymorph may have the following peaks. (Intensities may vary due to preferred orientation).

| D-space, Angstroms | Intensity, I/I₀, % |
|---|---|
| 12.25 | 43 |
| 11.28 | 28 |
| 8.78 | 60 |
| 8.17 | 61 |
| 7.73 | 81 |
| 7.43 | 28 |
| 6.77 | 77 |
| 6.32 | 94 |
| 6.11 | 46 |
| 5.61 | 34 |
| 5.32 | 30 |
| 5.13 | 92 |
| 4.98 | 100 |
| 4.88 | 65 |
| 4.85 | 69 |
| 4.71 | 38 |
| 4.63 | 42 |
| 4.55 | 49 |
| 4.39 | 57 |
| 4.32 | 45 |
| 4.14 | 49 |
| 4.03 | 38 |
| 3.97 | 41 |
| 3.87 | 90 |
| 3.69 | 31 |
| 3.64 | 72 |
| 3.49 | 87 |
| 3.46 | 37 |
| 3.38 | 39 |
| 3.36 | 33 |
| 3.23 | 38 |
| 3.16 | 26 |
| 3.13 | 31 |
| 2.98 | 24 |
| 2.95 | 27 |
| 2.91 | 37 |
| 2.87 | 20 |
| 2.84 | 22 |
| 2.80 | 25 |
| 2.79 | 23 |
| 2.57 | 19 |
| 2.34 | 20 |
| 2.19 | 20 |

The fexofenadine hydrochloride polymorph of the invention may be obtained by suspending 4-[4[4-(hydroxy diphenyl methyl)-1-piperidenyl]-1-hydroxy butyl]-α,α-dimethylphenyl acetic acid (fexofenadine) in pentanone. The suspension is concentrated whilst maintaining the Karl Fischer of the suspension below 1%, allowed to cool and HCl added in alcohol. On acid addition, a precipitate forms and after further cooling, this is collected by filtration, washed and dried. The resulting solid is resuspended in pentanone, the suspension refluxed and the polymorph of the invention isolated by filtration upon cooling.

Thus, the invention further provides a process for the preparation of a fexofenadine hydrochloride polymorph comprising:
I) mixing fexofenadine in pentanone to form a suspension;
II) heating said suspension so that an amount of pentanone is distilled off whilst maintaining the Karl Fischer of the suspension below 1% to form a slurry;
III) contacting said slurry with HCl in alcohol; and
IV) isolating the resulting precipitate and refluxing the same in pentanone.

The invention thus further provides a fexofenadine hydrochloride polymorph obtainable by, e.g. obtained by, a process as hereinbefore described.

The pentanone employed in the process of the invention should preferably be 3-pentanone. It is believed however, that other solvents such as 2-pentanone, and other ketones may also be employed. Typically, approximately 10 ml of pentanone per 1 g of fexofenadine should be initially mixed.

During the distillation step (II), it is preferred if at least 20%, e.g. at least 25%, such as approximately 30%, of the pentanone is distilled off to leave the slurry. Throughout this process the Karl Fischer of the suspension must be maintained below 1%, preferably below 0.5%, especially below 0.3%.

"Karl Fischer" refers to a conventional method for determining the content of water in solids and organic solvents. Thus, "Karl Fischer" is a measure of the water content of the suspension and is measured using standard means, see, e.g., the Skoog and West, "Fundamentals of Analytical Chemistry", 4th ed., 1982, pages 389-91, (ISBN 4-8337-0082-4).

The hydrochloric acid added to the slurry in step III is mixed with an alcohol. Preferably, the ratio of acid to alcohol should be in the range 1:1 to 1:2, especially around 2:3. It is also preferable to use a 2-3M solution of hydrochloric acid in alcohol. The alcohol of use is preferably ethanol although other alcohols such as propanol, methanol or isopropanol could be used. A slight molar excess of HCl (e.g. around 1.05:1 or 1.1:1) should be used in proportion to fexofenadine. Moreover, it is preferred if as little HCl/alcohol solution is used as possible since larger volumes of the acid/alcohol solution impedes precipitation. Hence, a more concentrated solution is preferred since less solution is required in order for the HCl to be in slight molar excess.

After addition of the acid precipitation occurs. It is believed that this is a fexofenadine hydrochloride pentanone solvate. The acidified mixture may be stirred to ensure full precipitation after which the mixture may be cooled to approximately 0°C. The precipitate may then be isolated by conventional techniques, e.g. filtration.

Conversion through to the polymorph of the invention is achieved by refluxing the precipitate (fexofenadine hydrochloride pentanone solvate) in pentanone, typically for 1 to 3, e.g. about 2 hours. On cooling the desired polymorph may be isolated by conventional techniques, e.g. filtration.

The fexofenadine polymorph described above may be formulated and employed in medical treatment as is well-known in the art. For example, fexofenadine may be employed as an antihistamine, antiallergy agent or bronchodilator and may be administered alone or in conjunction with other active agents. Pharmaceutical preparations of fexofenadine or its derivatives may take the form of tablets, capsules, powders, solutions, suspensions or emulsions, etc. These may be prepared using conventional pharmaceutical excipients or carriers.

The polymorph of the invention may be administered along with other polymorphs of fexofenadine or fexofenadine hydrochloride and the pharmaceutical composition of the invention covers this possibility. Thus, for example, the pharmaceutical composition of the invention may comprise 10%, preferably at least 20%, especially at least 30% of the fexofenadine polymorph of the invention.

Fexofenadine or its derivatives may be administered orally, parenterally, or across a mucous membrane. The skilled artisan is aware of other administration methods.

The amount of fexofenadine or its derivatives employed will vary depending on the nature of the patient but will be readily determined by the person skilled in the art.

The invention will now be further described with reference to the following non-limiting examples and Figures.

### BRIEF DESCRIPTION OF THE DRAWINGS

Figure 1 depicts the DSC curve of the fexofenadine hydrochloride polymorph of the invention showing a melt endotherm onset of 196.56°C.
Figures 2a to 2c depict the IR spectrum of the fexofenadine hydrochloride polymorph of the invention.
Figure 3 depicts the X-ray diffraction pattern of the fexofenadine hydrochloride polymorph of the invention.

### EXAMPLES

### Example 1

4-[4[4-(hydroxy diphenyl methyl)-1-piperidenyl]-1-hydroxy butyl]-α,α-dimethylphenyl acetic acid (330.0 g, 0.598 mol) was stirred with 3-pentanone (3000 ml). The resulting suspension was heated to distill off 900 ml of the 3-pentanone whilst maintaining the Karl Fischer of the suspension below 1%. The Karl Fischer of the suspension was initially 0.28%, after distillation, the Karl Fischer of the slurry was 0.13%. If the Karl Fischer is greater than 1% then more 3-pentanone should be added and distillation restarted.

The resulting thick slurry was cooled and subsequently treated with a 2.7 N solution of hydrochloric acid in ethanol (250 ml, 0.675 mol). The reaction was stirred and a precipitate observed. The solution was cooled to 0°C and stirred for one hour. The solid was collected by filtration on a sintered glass funnel, washed with 3-pentanone (450 ml) and vacuum dried at 40 to 50°C.

A suspension of this solid (328.8 g, Loss On Drying approx 10%) was heated at reflux with 3-pentanone (3619 ml). Complete conversion into the fexofenadine hydrochloride polymorph was checked by IR. The suspension was cooled to 0°C and stirred for 1 hour. The solid was collected by filtration on a sintered glass funnel, washed with 3-pentanone (329 ml) and vacuum dried at 40 to 50°C.

Yield 282 g of fexofenadine hydrochloride polymorph.

### Analytical data:

The solvent content, measured by gas chromatography was approximately 0.3% (3-pentanone).

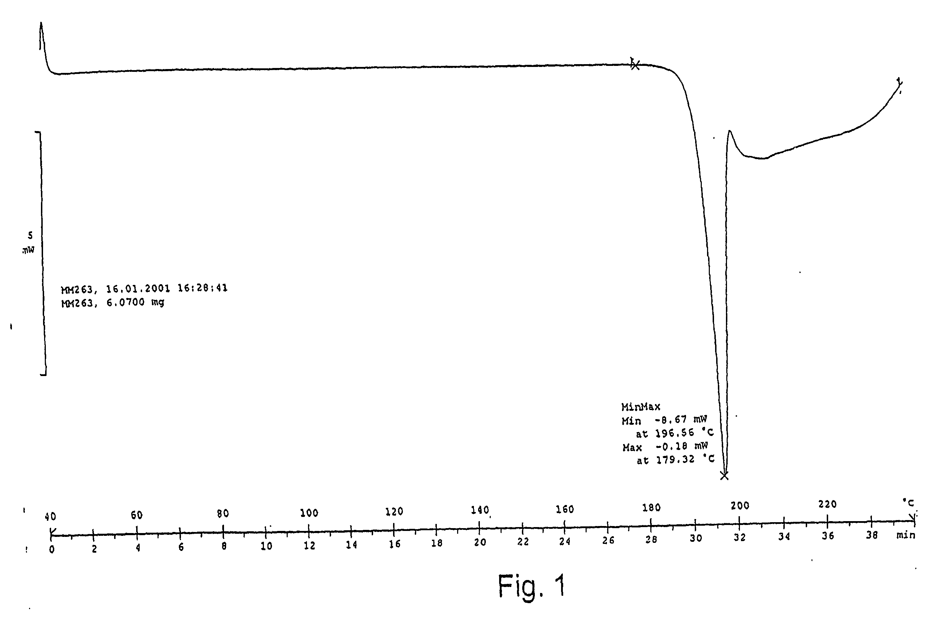

Melting point was determined using DSC (Metller Toledo STAR^{e} System, DSC821^{e}module). The temperature was taken from 40°C to 240°C, 5°C/min. Melt endotherm onset 195-197°C. (See Figure 1)

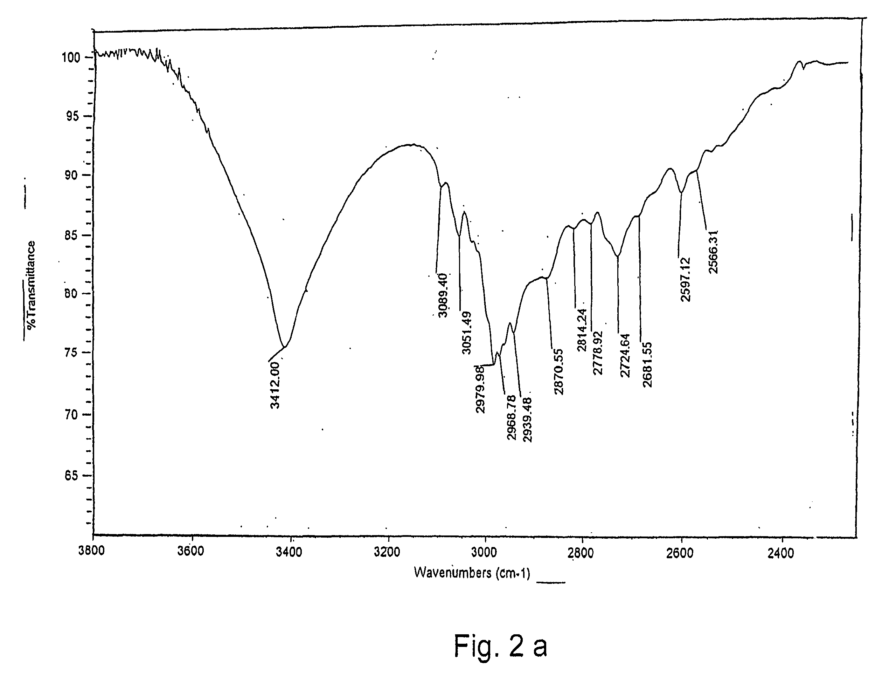

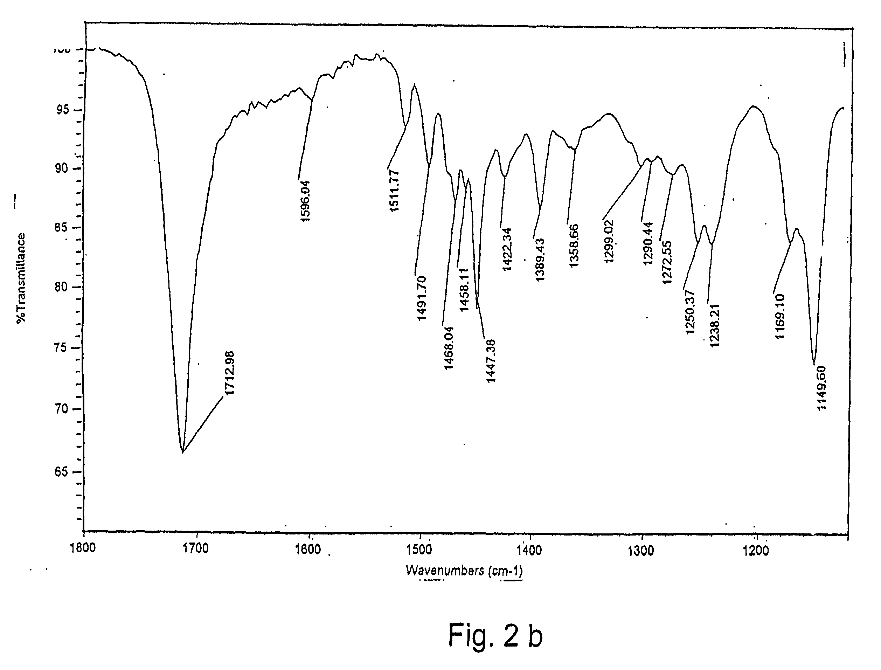

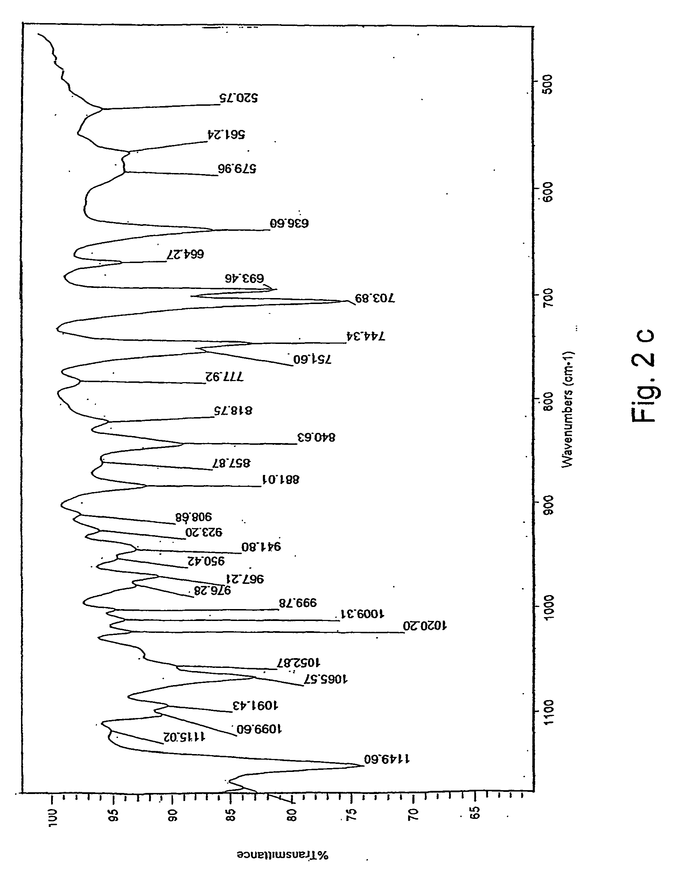

IR was measured using a Nicolet AVATAR 320. The results are presented in Figures 2a-c.

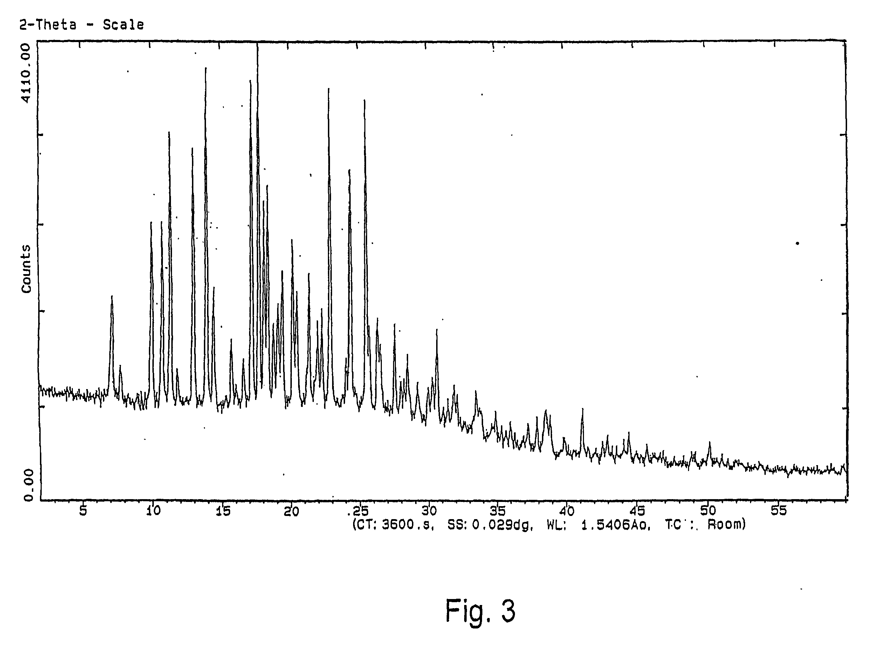

The X-ray powder diffraction pattern of the product was measured using a Debye-Scherrer INEL CPS-120, radiation K_{α}1 Cu(λ=1.5406Å). The results are presented below and in Figure 3.

| -N- | 2 theta | ---d--- | ---Cps--- | ---%--- |
|---|---|---|---|---|
| 1 | 7.211 | 12.2491 | 0.50 | 43.36 |
| 2 | 7.828 | 11.2842 | 0.32 | 28.47 |
| 3 | 10.062 | 8.7833 | 0.69 | 60.46 |
| 4 | 10.814 | 8.1745 | 0.69 | 60.66 |
| 5. | 11.435 | 7.7316 | 0.92 | 80.51 |
| 6 | 11.895 | 7.4338 | 0.32 | 27.98 |
| 7 | 13.057 | 6.7749 | 0.88 | 77.06 |
| 8 | 13.995 | 6.3228 | 1.08 | 94.45 |
| 9 | 14.485 | 6.1098 | 0.52 | 45.50 |
| 10 | 15.358 | 5.7646 | 0.26 | 22.48 |
| 11 | 15.793 | 5.6068 | 0.39 | 34.33 |
| 12 | 16.108 | 5.4977 | 0.28 | 24.96 |
| 13 | 16.651 | 5.3198 | 0.34 | 30.19 |
| 14 | 17.284 | 5.1264 | 1.05 | 91.63 |
| 15 | 17.803 | 4.9782 | 1.14 | 100.00 |
| 16 | 18.168 | 4.8788 | 0.75 | 65.40 |
| 17 | 18.446 | 4.8060 | 0.79 | 68.98 |
| 18 | 18.842 | 4.7057 | 0.43 | 37.66 |
| 19 | 19.171 | 4.6258 | 0.48 | 41.97 |
| 20 | 19.489 | 4.5509 | 0.56 | 49.42 |
| 21 | 20.228 | 4.3863 | 0.65 | 56.67 |
| 22 | 20.533 | 4.3218 | 0.51 | 44.67 |
| 23 | 21.210 | 4.1855 | 0.32 | 28.44 |
| 24 | 21.439 | 4.1412 | 0.56 | 48.76 |
| 25 | 22.053 | 4.0273 | 0.43 | 38.05 |
| 26 | 22.354 | 3.9738 | 0.47 | 40.78 |
| 27 | 22.938 | 3.8739 | 1.03 | 89.88 |
| 28 | 24.114 | 3.6876 | 0.35 | 30.58 |
| 29 | 24.404 | 3.6444 | 0.83 | 72.41 |
| 30 | 24.712 | 3.5997 | 0.27 | 24.01 |
| 31 | 25.516 | 3.4881 | 1.00 | 87.42 |
| 32 | 25.737 | 3.4586 | 0.43 | 37.30 |
| 33 | 26.345 | 3.3802 | 0.44 | 38.83 |
| 34 | 26.546 | 3.3550 | 0.38 | 33.31 |
| 35 | 27.586 | 3.2309 | 0.43 | 37.64 |
| 36 | 28.018 | 3.1820 | 0.29 | 25.62 |
| 37 | 28.256 | 3.1557 | 0.30 | 26.28 |
| 38 | 28.512 | 3.1280 | 0.36 | 31.34 |
| 39 | 28.658 | 3.1124 | 0.26 | 22.87 |
| 40 | 29.227 | 3.0530 | 0.29 | 25.26 |
| 41 | 30.010 | 2.9751 | 0.28 | 24.16 |
| 42 | 30.312 | 2.9462 | 0.30 | 26.69 |
| 43 | 30.664 | 2.9131 | 0.42 | 36.59 |
| 44 | 31.103 | 2.8730 | 0.23 | 20.07 |
| 45 | 31.428 | 2.8441 | 0.25 | 21.97 |
| 46 | 31.887 | 2.8042 | 0.29 | 25.04 |
| 47 | 32.085 | 2.7873 | 0.26 | 22.68 |
| 48 | 33.527 | 2.6707 | 0.27 | 23.26 |
| 49 | 33.808 | 2.6491 | 0.23 | 20.05 |
| 50 | 34.637 | 2.5876 | 0.19 | 16.67 |
| 51 | 34.946 | 2.5654 | 0.22 | 19.32 |
| 52 | 35.350 | 2.5370 | 0.18 | 16.18 |
| 53 | 35.680 | 2.5143 | 0.18 | 15.43 |
| 54 | 36.007 | 2.4922 | 0.20 | 17.20 |
| 55 | 36.327 | 2.4710 | 0.17 | 14.70 |
| 56 | 36.965 | 2.4298 | 0.16 | 14.09 |
| 57 | 37.286 | 2.4096 | 0.19 | 16.64 |
| 58 | 37.932 | 2.3700 | 0.21 | 18.18 |
| 59 | 38.523 | 2.3350 | 0.22 | 19.68 |
| 60 | 38.680 | 2.3259 | 0.21 | 17.98 |
| 61 | 38.897 | 2.3134 | 0.21 | 18.44 |
| 62 | 39.911 | 2.2570 | 0.16 | 13.89 |
| 63 | 41.190 | 2.1898 | 0.23 | 20.12 |
| 64 | 41.594 | 2.1695 | 0.14 | 11.97 |
| 65 | 42.111 | 2.1440 | 0.13 | 11.78 |
| 66 | 42.649 | 2.1182 | 0.15 | 12.87 |
| 67 | 42.981 | 2.1026 | 0.16 | 14.23 |
| 68 | 43.289 | 2.0884 | 0.14 | 12.24 |
| 69 | 43.635 | 2.0726 | 0.14 | 12.19 |
| 70 | 44.117 | 2.0510 | 0.15 | 13.50 |
| 71 | 44.500 | 2.0343 | 0.17 | 14.94 |
| 72 | 45.025 | 2.0118 | 0.13 | 10.97 |
| 73 | 45.377 | 1.9970 | 0.12 | 10.27 |
| 74 | 45.750 | 1.9816 | 0.14 | 12.41 |
| 75 | 46.241 | 1.9617 | 0.12 | 10.27 |
| 76 | 46.683 | 1.9441 | 0.12 | 10.51 |
| 77 | 46.903 | 1.9355 | 0.13 | 11.09 |
| 78 | 47.657 | 1.9066 | 0.11 | 9.81 |
| 79 | 48.356 | 1.8807 | 0.12 | 10.07 |
| 80 | 48.900 | 1.8611 | 0.12 | 10.66 |
| 81 | 49.154 | 1.8520 | 0.12 | 10.92 |
| 82 | 49.703 | 1.8328 | 0.11 | 9.54 |
| 83 | 50.150 | 1.8175 | 0.15 | 12.87 |

## Claims

1. A fexofenadine hydrochloride polymorph having the following X-ray powder diffraction pattern obtained using K_{α}1 Cu(λ=1.5406Å) radiation
| D-space, Angstroms |
|---|
| 12.25 |
| 11.28 |
| 8.78 |
| 8.17 |
| 7.73 |
| 6.77 |
| 6.32 |
| 6.11 |
| 5.61 |
| 5.32 |
| 5.13 |
| 4.98 |
| 4.88 |
| 4.85 |
| 4.32 |
| 3.87 |
| 3.64 |
| 3.49 |
| 3.38 |
| 3.23 |
| 2.91 |
| 2.80 |

2. A fexofenadine hydrochloride polymorph according to claim 1 having the characteristic IR peaks IR νₘₐₓ(cm⁻¹)(KBr): 3412, 1713, 1250, 1238, 1150, 1091, 751, 744, 704, 693.

3. A pharmaceutical composition comprising the fexofenadine hydrochloride polymorph as claimed in claim 1 or 2 along with one or more pharmaceutical carriers/exdpients.

4. A fexofenadine hydrochloride polymorph as claimed in claim 1 or 2 for use in medicine.

5. The use of the fexofenadine hydrochloride polymorph as claimed in claim 1 or 2 in the manufacture of an medicament for use as an antihistamine, antiallergy agent or bronchodilator.

6. A process for the preparation of a fexofenadine hydrochloride polymorph as defined in claim 1 comprising:
I) mixing fexofenadine in pentanone to form a suspension;
II) heating said suspension so that an amount of pentanone is distilled off whilst maintaining the Karl Fischer of the suspension below 1% to form a slurry;
III) contacting said slurry with hydrogen chloride in alcohol; and
IV) isolating the resulting precipitate and refluxing the same in pentanone.

7. A fexofenadine hydrochloride polymorph obtainable by a process as claimed in claim 6.

## Patentansprüche

1. Fexo-Fenadinchlorhydrat-Polymorph mit folgendem gewonnenen Pulverröntgenbeugungsmuster, unter Verwendung von K_{α}1 Cu(λ=1,5406Å)-Strahlung
| D-Abstand, Ångström |
|---|
| 12,25 |
| 11,28 |
| 8,78 |
| 8,17 |
| 7,73 |
| 6,77 |
| 6,32 |
| 6,11 |
| 5,61 |
| 5,32 |
| 5,13 |
| 4,98 |
| 4,88 |
| 4,85 |
| 4,32 |
| 3,87 |
| 3,64 |
| 3,49 |
| 3,38 |
| 3,23 |
| 2,91 |
| 2,80 |

2. Fexo-Fenadinchlorhydrat-Polymorph gemäss Anspruch 1 mit den charakteristischen IR Peaks IR νₘₐₓ(cm⁻¹)(KBr): 3412, 1713, 1250, 1238, 1150, 1091, 751, 744, 704, 693.

3. Pharmazeutische Zusammensetzung, umfassend Fexo-Fenadinchlorhydrat-Polymorph, wie in den Ansprüchen 1 oder 2 beansprucht, zusammen mit einem oder mehreren farmazeutischen Trägern/Hilfsstoffen.

4. Fexo-Fenadinchlorhydrat-Polymorph, wie in den Ansprüchen 1 oder 2 beansprucht, zur Verwendung in Arzneimitteln.

5. Verwendung des Fexo-Fenadinhydrochlorid-Polymorphs, wie in den Ansprüchen 1 oder 2 beansprucht, bei der Herstellung eines Medikaments zur Verwendung als ein Antihistaminikum, Antiallergiemittel oder Bronchodilatator.

6. Verfahren zur Herstellung eines Fexo-Fenadinchlorhydratpolymorphs, wie in Anspruch 1 definiert, umfassend:
I) Mischen von Fexo-Fenadin in Pentanon zur Bildung einer Suspension;
II) Erwärmen der Suspension, so dass eine Menge von Pentanon ausdestilliert ist, während der Karl Fischer der Suspension unter 1% gehalten wird, um eine Aufschlämmung zu bilden;
III) Kontaktieren der Ausschlämmung mit Chlorwasserstoff in Alkohol; und
IV) Isolieren der entstandenen Präzipitat und Refluxieren derselben in Pentanon.

7. Fexo-Fenadinchlorhydrat-Polymorph, das durch ein Verfahren, wie in Anspruch 6 beansprucht, gewonnen werden kann.

## Revendications

1. Polymorphe de fexofénadine-hydrochloride ayant le motif de diffraction des rayons X sur poudre suivant, obtenu en utilisant la radiation K_{α}1 Cu(λ=1,5406Å)
| Espace D, Ångstroms |
|---|
| 12,25 |
| 11,28 |
| 8,78 |
| 8,17 |
| 7,73 |
| 6,77 |
| 6,32 |
| 6,11 |
| 5,61 |
| 5,32 |
| 5,13 |
| 4,98 |
| 4,88 |
| 4,85 |
| 4,32 |
| 3,87 |
| 3,64 |
| 3,49 |
| 3,38 |
| 3,23 |
| 2,91 |
| 2,80 |

2. Polymorphe de fexofénadine-hydrochloride selon la revendication 1 ayant les pics IR caractéristiques IR νₘₐₓ(cm⁻¹)(KBr): 3412, 1713, 1250, 1238, 1150, 1091, 751, 744, 704, 693.

3. Composition pharmaceutique comprenant le polymorphe de fexofénadine-hydrochloride selon la revendication 1 ou 2 avec un ou plusieurs porteurs/excipients pharmaceutiques.

4. Polymorphe de fexofénadine-hydrochloride selon la revendication 1 ou 2 pour l'utilisation dans la médicine.

5. Utilisation du polymorphe de fexofénadine-hydrochloride selon la revendication 1 ou 2 dans la manufacture d'un médicament pour l'utilisation comme un antihistaminique, un agent anti-allergique ou un bronchodilateur.

6. Procédé de préparation d'un polymorphe de fexofénadine-hydrochloride comme défini dans la revendication 1, comprenant:
I) mélange du fexofénadine dans du pentanone pour former une suspension;
II) chauffage de la suspension pour séparer par distillation une quantité de pentanone en maintenant le Karl Fischer de la suspension en-dessous de 1% pour former une suspension;
III) mise en contact de la suspension avec du chlorure d'hydrogène dans de l'alcool; et
IV) isolation du précipitat résultant et reflux de ce dernier dans du pentanone.

7. Polymorphe de fexofénadine-hydrochloride qui peut être obtenu par un procédé selon la revendication 6.
